# EUROPÄISCHE PATENTANMELDUNG

(11) **EP 2 561 811 A1**
(43) Veröffentlichungstag der Anmeldung: **27.02.2013**
(21) Anmeldenummer: 12178253.6
(22) Anmeldetag: 27.07.2012
(51) Int. Cl.: A61B 5/11

(54) **Vergleich der rechts- und linksventrikulären Kontraktion mittels Akzelerometer in einer herznahen Arterie**

(30) Priorität: 23.08.2011 US 201161526280 P
(71) Anmelder: BIOTRONIK SE & Co. KG, 12359 Berlin (DE)
(72) Erfinder: Kirchner, Jens, 91052 Erlangen (DE); Vollkron, Michael, 3021 Pressbaum (AT); Skerl, Olaf, 18209 Bad Doberan (DE)
(74) Vertreter: Lindner-Vogt, Karin L.

(57) **Zusammenfassung**

Die Erfindung betrifft ein implantierbares medizinisches Gerät mit einem mehrachsigen Beschleunigungssensor und einer mit diesem verbundenen Auswerteeinheit. Die Auswerteinheit ist ausgebildet, ein Akzelerometerausgangssignal des Beschleunigungssensors auszuwerten, indem die Auswerteeinheit
- das Akzelerometerausgangssignal in zumindest zwei Signalkomponenten aufspaltet, von denen wenigstens zwei in Zusammenhang mit einer rechts- bzw. linksventrikulären Kontraktion eines Herzens stehen;
- in den wenigstens zwei Signalkomponenten Ereignisse detektiert und/oder Signalmerkmale bestimmt; und
- zumindest eine Kenngröße K unter Auswertung der wenigstens zwei Signalkomponenten und der aus diesen bestimmten Ereignisse und/oder Signalmerkmale bestimmt.

## Beschreibung

Die Erfindung betrifft ein implantierbares medizinisches Gerät mit einem mehrachsigen Beschleunigungssensor und einer mit diesem verbundenen Auswerteeinheit, die ausgebildet ist, ein Akzelerometerausgangssignal des Beschleunigungssensors auszuwerten. Solche Geräte sind beispielsweise in Form von implantierbaren ratenadaptiven Herzschrittmachern bekannt, bei denen ein Beschleunigungssensor zum Erfassen körperlicher Aktivität eines Herzschrittmacher-Trägers dient.

Ziel der Erfindung ist es, ein Gerät für das Monitoring von Herzfunktionen und/oder die Steuerung von Therapiegeräten zu schaffen, das neue Monitoring- und/oder Steuerungsoptionen eröffnet und so beispielsweise eine Detektion krankhafter Veränderungen im ventrikulären Kontraktionsverhalten sowie ein Monitoring der Hämodynamik im allgemeinen erlaubt.

Erfindungsgemäß wird dieses Ziel durch ein implantierbares medizinisches Gerät mit einem mehrachsigen Beschleunigungssensor und einer mit diesem verbundenen Auswerteeinheit gelöst, wobei die Auswerteinheit ausgebildet ist, ein Akzelerometerausgangssignal des Beschleunigungssensors auszuwerten, indem die Auswerteeinheit
- aus dem Akzelerometerausgangssignal zumindest zwei Signalkomponenten A1 und A2 extrahiert, von denen jeweils eine in Zusammenhang mit einer rechtsventrikulären Kontraktion und eine in Zusammenhang mit einer linksventrikulären Kontraktion eines Herzens steht (Signalkomponente A1 und Signalkomponente A2);
- in den wenigstens zwei Signalkomponenten Ereignisse und/oder Signalmerkmale, wie beispielsweise Spitzenamplituden, Intervalle, Frequenzen etc., bestimmt (beispielsweise indem die Auswerteinheit die beiden Signalkomponenten jeweils entsprechend auswertet), und
- zumindest eine Kenngröße K unter Auswertung der wenigstens zwei Signalkomponenten und der aus diesen bestimmten Ereignissen und/oder Signalmerkmalen bestimmt.

Es ergibt sich somit ein Gerät, das vorzugsweise
- ein 3D-Akzelerometer aufweist, welches im Einsatzfall an einer geeigneten Position in der Pulmonalarterie oder der Aorta in Herznähe implantiert ist und zur Messung mehrachsiger Beschleunigung und Erzeugung eines diese widerspiegelnden Akzelerometerausgangssignals A dient; sowie
- eine Auswerteeinheit, die das folgende Verfahren ausführt:
   (a) Aufspaltung des Akzelerometerausgangssignals A in zumindest zwei Signalkomponenten, von denen mindestens zwei in Zusammenhang mit der rechts- bzw. der linksventrikulären Kontraktion stehen (Signalkomponenten A1 und A2);
   (b) Bestimmung geeigneter Ereignisse und/oder Merkmale aus den Signalen A1 und A2; und
   (c) Bestimmung zumindest einer Kenngröße K unter Verwendung mindestens der Signalkomponenten A1 und A2 und der in (b) bestimmten Ereignisse/Merkmale.

Bevorzugt ist K das Verhältnis zwischen zwei Amplitudenwerten aus A1 und A2, z.B. der Maximalamplituden innerhalb eines Herzzyklus. Ebenso bevorzugt spiegelt K den Zeitversatz zwischen zwei Ereignissen aus A1 bzw. A2 wieder, z.B. eine Größe, die mit dem VV-Delay zusammenhängt.

Das erfindungsgemäße Gerät bietet den Vorteil, dass mit einem Beschleunigungssensor beide Ventrikel überwacht werden können und die abgeleiteten Informationen spezifisch für eine jeweilige Herzhälfte sind.

Dies stellt einen großen Vorteil gegenüber bekannten Lösungen dar, die einen Vergleich von linkem und rechtem Herz mit mehreren getrennten Sensoren für beispielsweise eine Analyse von Impedanz, Blutdruck, Herztönen, Ballistokardiogramm etc. vorsehen.

Die genannten bekannten Methoden beschränken sich entweder auf das Verhalten des gesamten Herzens (Herztöne, Ballistokardiogramm) bzw. eines Teils des Herzen (Impedanz) oder, falls sie innerhalb einer Herzkammer/Arterie aufgezeichnet werden, auf eine einzige Herzhälfte (endokardiale Beschleunigungen, Blutdruck). Ein Vergleich der beiden Herzhälften gegeneinander ist mit diesem Ansatz nicht möglich.

Für eine Gegenüberstellung der beiden Herzhälften werden i. d. R. zwei räumlich getrennte Sensoren verwendet, die z.B. in den Ventrikeln oder den angrenzenden Arterien platziert werden. Im Gegensatz zu einem 1-Sensor-System sind bei einem solchen 2-Sensoren-System Energiebedarf, Fehleranfälligkeit sowie die Belastung für Arzt und Patient deutlich erhöht.

In Bezug auf das hier vorgestellte Gerät ist es bevorzugt, wenn der Beschleunigungssensor ein 3D-Akzelerometer ist, das zum herznahen Platzieren in der Pulmonalarterie oder der Aorta ausgebildet ist.

Die Auswerteinheit ist weiterhin ausgebildet, die wenigstens zwei Signalkomponenten anhand von Richtungskomponenten des Akzelerometerausgangssignals zu bestimmen.

Der Bereich der Pulmonalarterie im und kurz hinter dem Truncus pulmonalis, insbesondere der Ansatz der Arteria pulmonaris dextra, zeichnet sich durch eine enge Nachbarschaft zur Aorta aus. Hier überlagern sich während einer Herzkontraktion Bewegungsanteile aus dem linken und rechten Ventrikel. Mit Hilfe eines 3D-Akzelerometers lassen sich diese Komponenten aufzeichnen, anschließend trennen und vergleichen. Die daraus berechneten Größen dienen zur Detektion und Überwachung ventrikelspezifischer krankhafter Veränderung des Kontraktionsverhaltens.

Aufgrund der unterschiedlichen Anatomie von Aorta und Pulmonalarterie sind die Richtungskomponenten der durch die Blutpulsation verursachten Beschleunigungen und des diese widerspiegelnden Akzelerometerausgangssignals unterschiedlich. Des Weiteren entsteht durch die typische Verzögerung zwischen den Kontraktionen von linkem und rechtem Ventrikel ein Zeitversatz zwischen den Komponenten. Diese beiden Effekte erlauben der Auswerteeinheit die Trennung von Beschleunigungskomponenten aus der Aorta und der Pulmonalarterie.

Die Auswerteinheit ist außerdem vorzugsweise dazu ausgebildet, die Kenngröße K aus dem Verhältnis zwischen zwei Amplitudenwerten als jeweiliges Signalmerkmal aus den wenigstens zwei Signalkomponenten zu bestimmen, insbesondere aus dem Verhältnis der Maximalamplituden innerhalb eines Herzzyklus.

Alternativ oder zusätzlich kann die Auswerteinheit dazu ausgebildet sein, die Kenngröße K aus einem jeweiligen Zeitversatz zwischen zwei einander entsprechenden Ereignissen in den wenigstens zwei Signalkomponenten zu bestimmen, so dass die Kenngröße K insbesondere eine Größe ist, die mit einer interventrikulären Verzögerungszeit (VV-Delay) zusammenhängt.

Vorzugsweise ist die Auswerteeinheit zusätzlich zur Ableitung zumindest einer weiteren Signalkomponente (Signalkomponente A3) in Schritt (a) und Bestimmung der Kenngröße K in Abhängigkeit eines aus der Signalkomponente A3 abgeleiteten Wertes ausgebildet. Hierbei ist die Signalkomponente A3 vorzugsweise eine die Aktivität des Patienten widerspiegelnde Signalkomponente und der aus ihr abgeleitete Wert die mittlere Aktivität in einem vordefinierten Zeitintervall. Dabei können bestimmte Bereiche des Aktivitätssignals zu Aktivitätslevels zusammengefasst werden.

Alternativ oder zusätzlich kann die Auswerteinheit ausgebildet sein, zusätzlich zumindest eine weitere Signalkomponente (Signalkomponente A3) in Schritt (a) aus dem Akzelerometerausgangssignal abzuleiten und die Kenngröße K (nur) zu solchen Zeitpunkten zu bestimmen, in denen eine aus der Signalkomponente A3 abgeleitete Größe in einem vordefinierten Wertebereich liegt.

Weiterhin kann die Auswerteinheit ausgebildet sein, zusätzlich eine Größe J aus den Signalkomponenten A1 und/oder A2 zu bestimmen und die Kenngröße K in Abhängigkeit der Größe J zu bestimmen. Hierbei ist die Größe J vorzugsweise eine Herzrate eines Patienten. Auch in diesem Fall kann die Auswerteinheit dazu ausgebildet sein, die Größe J aus den Signalkomponenten A1 und/oder A2 zu bestimmen und die Kenngröße K (nur) zu solchen Zeitpunkten zu bilden, in denen J in einem vordefinierten Wertebereich liegt.

Vorzugsweise ist das implantierbare Gerät zusätzlich mit einem weiteren Sensor zur Messung eines weiteren Signals B ausgestattet und die Auswerteinheit ist zur Bestimmung der Kenngröße K in Abhängigkeit einer aus dem Signal B abgeleiteten Größe ausgebildet. Der weitere Sensor ist dabei vorzugsweise ein Blutdruck- und/oder ein Impedanzsensor, so dass das Signal B einen Blutdruck oder eine Impedanz widerspiegelt. Die aus dem Signal B abgeleitete Größe entspricht vorzugsweise einem Mittelwert, einem diastolischen oder einem systolischen Wert.

Zusätzlich oder alternativ kann das implantierbare Gerät mit einem weiteren Sensor zur Messung des weiteren Signals B ausgestattet sein und die Auswerteeinheit zur Bestimmung der Kenngröße K zu solchen Zeitpunkten, in denen eine aus dem Signal B abgeleitete Größe in einem vordefinierten Wertebereich liegt.

Gemäß einer weiteren bevorzugten Ausführungsvariante ist das Gerät dazu ausgebildet, Kenngrößen der Atmung zu bestimmen, insbesondere Frequenz und Amplitude. Bevorzugte Realisierungen sind die Bestimmung des Grades von Kurzatmigkeit bei gegebener physischer Belastung sowie die Detektion von Schlafapnoe.

Gemäß einer weiteren bevorzugten Ausführungsvariante ist das implantierbare Gerät zusätzlich zum Messen einer Sensorgröße, die in Zusammenhang mit der elektrischen Erregung des Herzens steht, und Ableitung einer Größe, die in Zusammenhang mit der elektromechanischen Kopplung steht, ausgebildet.

Vorzugsweise weist das implantierbare Gerät eine Steuereinheit auf, die ausgebildet ist, die Kenngröße K zur Detektion von Arrhythmien zu verwenden und Therapieeinheiten des implantierbaren medizinischen Gerätes entsprechend zu steuern. Bevorzugt ist die Verwendung der Kenngröße K als Schockkriterium bei Defibrillatoren, als Trigger für antitachykardes Pacing oder als reine Monitoring-Größe (Anzahl und Dauer bestimmter Tachykardien).

Gemäß einer weiteren bevorzugten Ausführungsvariante ist das implantierbare Gerät dazu ausgebildet, die Kenngröße K dazu zu verwenden, die Hämodynamik eines Patienten zu überwachen und insbesondere das Auftreten und die Verschlechterung von Herzkrankheiten, wie Herzinsuffizienz (CHF), zu detektieren. Bevorzugte Realisierungen sind die Integration in einen Algorithmus zur Prädiktion kardialer Dekompensationen, die Anzeige in einem externen Monitoring-System für den behandelnden Arzt, die Generierung eines Alarms bei Überschreiten eines Schwellwertes.

Gemäß einer weiteren bevorzugten Ausführungsvariante ist das implantierbare Gerät dazu ausgebildet, die Kenngröße K dazu zu verwenden, die Wirkung eines Medikaments sowie die tatsächliche Einnahme durch den Patienten zu überwachen.

Vorzugsweise weist das implantierbare Gerät eine Steuereinheit auf, die ausgebildet ist, in Abhängigkeit der Kenngröße K Einstellungen des implantierbaren medizinischen Geräts anzupassen. Bevorzugte Realisierungen sind die Optimierung von Pacing-Parametern in einem Schrittmacher/Defibrillator (z.B. AV-/VV-Delay) oder die Anpassung der Dosierung bei automatischer Medikamentengabe.

Insbesondere ist es bevorzugt, wenn das implantierbare Gerät ein Herzstimulator, insbesondere ein biventrikulärer Herzschrittmacher oder Defibrillator/Kardioverter ist.

Die Erfindung soll nun anhand von Ausführungsbeispielen mit Bezug auf die Figuren näher erläutert werden. Die Figuren zeigen Folgendes:
- **Fig. 1**: zeigt als implantierbares medizinisches Gerät einen implantierbaren Herzstimulator und daran angeschlossene implantierbare Elektrodenleitungen.
- **Fig. 2**: zeigt ein implantierbares medizinisches Gerät als Herzmonitor mit daran angeschlossenem, in der Pulmonalarterie platziertem mehrachsigen Beschleunigungssensor.
- **Fig. 3**: zeigt beispielhaft einige Komponenten eines erfindungsgemäßen implantierbaren medizinischen Gerätes, wie es beispielsweise in Figur 1 oder 2 dargestellt ist.
- **Fig. 4**: zeigt die Anatomie des Herzens mit angrenzenden Blutgefäßen sowie der Richtung des Blutflusses. Eingezeichnet ist weiterhin die Ebene, in der der Truncus pulmonalis und die Pulmonalarterien liegen.
- **Fig. 5**: zeigt den zu erwartenden Verlauf des Akzelerometersignals in der Schnittebene durch Truncus pulmonalis und die Arteria pulmonaris dextra (siehe Figur 4).
- **Fig. 6**: zeigt die Anatomie des Herzens mit angrenzenden Blutgefäßen sowie der Richtung des Blutflusses. Eingezeichnet ist weiterhin die Ebene, in der die Aorta liegt.
- **Fig. 7**: zeigt den zu erwartenden Verlauf des Akzelerometersignals in der Schnittebene durch den Aortenbogen (siehe Figur 6). Diese Ebene ist in etwa senkrecht zu der Ebene in Figur. 4;
- **Fig. 8**: zeigt zu erwartende Signalkomponenten eines mehrachsigen Akzelerometerausgangssignals über der Zeit, die sich aus dem zu erwartenden Verlauf des Beschleunigungssignals in Richtung der x- bzw. y-Achse entsprechend den Figuren 5 und 7 ergeben. Das obere Signal entspricht der rechts-, das untere der linksventrikulären Kontraktion.
- **Fig. 9**: zeigt die Anatomie des Herzens mit angrenzenden Blutgefäßen sowie der Richtung des Blutflusses. Eingezeichnet ist weiterhin die Ebene entlang der Gravitation, in der die Pulmonalarterien liegen.

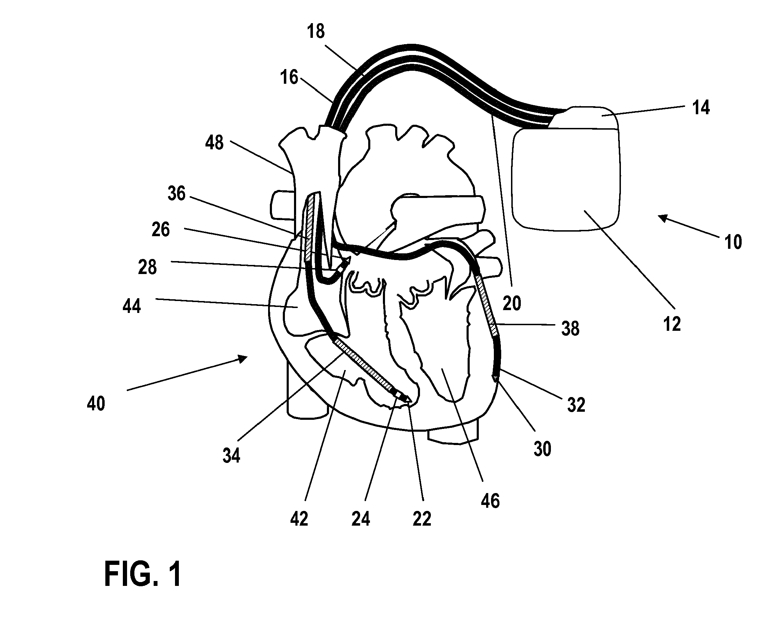

Fig. 1 zeigt einen Herzmonitor in Form eines implantierbaren medizinischen Gerätes, nämlich in Form eines implantierbaren Herzstimulators 10. Der Herzstimulator 10 ist als biventrikulärer Herzschrittmacher und Kardioverter/Defibrillator ausgebildet.

Der Herzstimulator 10 besitzt in an sich bekannter Weise ein Gehäuse 12 aus Metall, das auch als großflächige Elektrode dienen kann. An dem Gehäuse 12 ist ein sog. Header 14 aus Kunststoff angebracht, der als Anschlussgehäuse mit Steckboxen entsprechende Stecker von Elektrodenleitungen aufnehmen kann, um auf diese Weise Elektroden an den Elektrodenleitungen elektrisch mit elektrischen Komponenten im Inneren des Gehäuses 12 zu verbinden.

Im abgebildeten Fall sind insgesamt drei Elektrodenleitungen an dem Herzschrittmacher 10 angeschlossen, nämlich eine rechtsventrikuläre Elektrodenleitung 16, eine rechtsatriale Elektrodenleitung 18 und eine linksventrikuläre Elektrodenleitung 20. Jede dieser Elektrodenleitungen trägt an ihrem distalen Ende jeweils ein Paar vergleichsweise kleinflächiger Stimulations- und Sensingelektroden, konkret sind dies eine rechtsventrikuläre Spitzenelektrode RV-TIP 22, eine rechtsventrikuläre Ringelektrode RV-RING 24, eine rechtsatriale Spitzenelektrode RA-TIP 26 und eine rechtsatriale Ringelektrode RA-TIP 28, eine rechtsatriale Elektrodenleitung 18 und schließlich eine linksventrikuläre Spitzenelektrode RV-TIP 30 und eine linksventrikuläre Ringelektrode RV-TIP 32 am distalen Ende der linksventrikulären Elektrodenleitung 20.

Zum Zweck einer Defibrillationsschockabgabe sind außerdem noch vergleichsweise großflächige Defibrillationselektroden vorgesehen, die jeweils als Schockwendel ausgebildet sind. Konkret sind dies eine rechtsventrikuläre Schockelektrode RV-COIL 34, welche auf der rechtsventrikulären Elektrodenleitung 16 in der Nähe von deren distalem Ende angeordnet ist. Die rechtsventrikuläre Elektrodenleitung 16 trägt außerdem auch noch eine für die Platzierung der Vena cava vorgesehene proximale Schockelektrode VC-COIL 36. In der Nähe des distalen Endes der linksventrikulären Elektrodenleitung 20 trägt diese eine linksventrikuläre Schockelektrode RV-COIL 38.

Fig. 1 zeigt schematisch, wie die einzelnen Elektroden nach Implantation ungefähr im Herzen angeordnet sind. So zeigt Fig. 1 eine schematische Darstellung eines Herzens 40 mit seinem rechten Ventrikel 42, seinem rechten Atrium 44 und seinem linken Ventrikel 46. Außerdem ist ein Abschnitt der Vena cava superior 48 abgebildet.

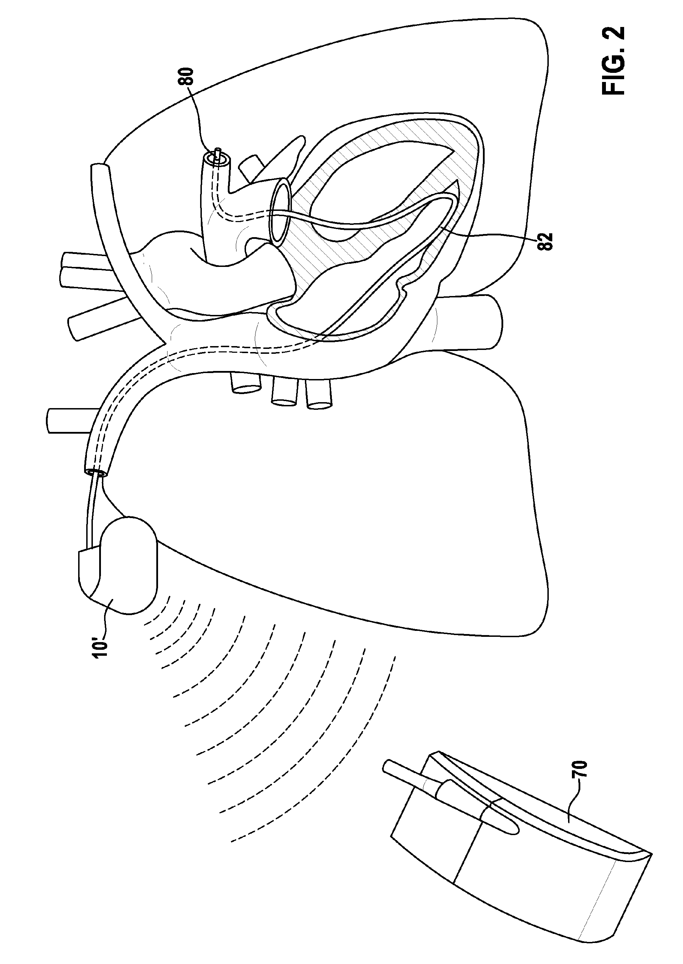

Figur 2 zeigt ein implantierbares medizinisches Gerät 10' als Herzmonitor. Der Einfachheit halber sind die in Fig. 1 dargestellten Elektrodenleitungen in der Darstellung weggelassen. Dafür ist zum einen ein externes Gerät 70 (Patientengerät), das zur drahtlosen Kommunikation mit dem implantierbaren medizinischem Gerät 10' dient, gezeigt. Vor allem aber zeigt Fig. 2 eine Beschleunigungssensoreinheit 80, die als mehrachsiger Beschleunigungssensor ausgebildet ist und über eine Anschlussleitung 82 mit dem implantierbaren medizinischem Gerät 10' verbunden ist. Die Beschleunigungssensoreinheit 80 ist in Herznähe in der Pulmonalarterie angeordnet, um dort mehrachsige Beschleunigungen aufzunehmen und ein entsprechendes Akzelerometerausgangssignal zu liefern.

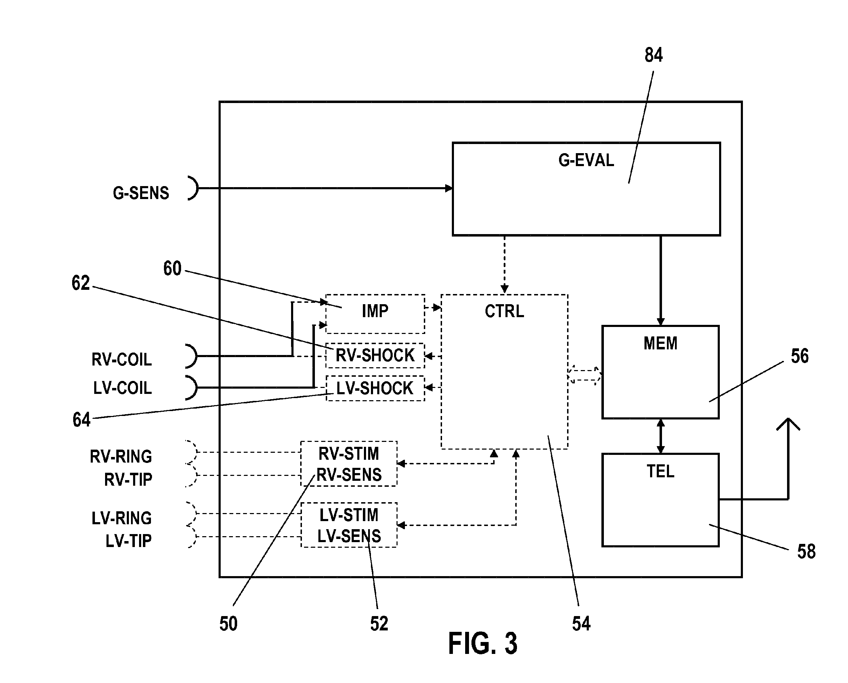

Figur 3 zeigt einige derjenigen elektrischen bzw. elektronischen Komponenten des Herzstimulators 10, die in dessen Gehäuse 12 angeordnet sind, nämlich eine rechtsventrikuläre Stimulationseinheit RV-STIM und eine rechtsventrikuläre Sensingeinheit RV-SENS, die schematisch dargestellt sind und mit einem gemeinsamen Bezugszeichen 50 versehen sind. Die rechtsventrikuläre Stimulationseinheit und die rechtsventrikuläre Sensingeinheit sind mit dem elektrischen Anschluss für die rechtsventrikuläre Ringelektrode RV-RING und die rechtsventrikuläre Spitzenelektrode RV-TIP verbunden. In gleicher Weise sind eine linksventrikuläre Stimulationseinheit LV-STIM und eine linksventrikuläre Sensingeinheit LV-SENS (gemeinsames Bezugszeichen 52) mit dem elektrischen Anschluss für die linksventrikuläre Spitzenelektrode LV-TIP und die linksventrikuläre Ringelektrode LV-RING verbunden. Die Stimulations- und Sensingeinheiten sind außerdem mit einer zentralen Steuereinheit CTRL 54 verbunden.

Die Steuereinheit ist außerdem mit einem Impedanzsensor 60 verbunden, der außerdem mit den Anschlüssen RV-Coil und LV-Coil verbunden ist, um über die Schockelektroden 34 und 36 einen unterschwelligen, gepulsten, bipolaren Messstrom einzuspeisen und die dabei über die beiden Schockelektroden 34 und 36 abfallende Spannung zu messen und so die Impedanz zu bestimmen.

Die Steuereinheit CTRL 54 ist mit einem Speicher MEM 56 verbunden, der seinerseits wiederum mit einer Telemetrieeinheit TEL 58 verbunden ist. Die Speichereinheit MEM 56 dient dazu, vom Herzstimulator 10 erfasste physiologische oder Betriebsdaten zwischenzuspeichern, wenn diese über die Telemetrie TEL 58 an ein externes Gerät gesendet werden sollen. Außerdem können in der Speichereinheit MEM 56 auch Parameter oder Programmdaten hinterlegt sein, auf die die Steuereinheit CTRL 54 zurückgreift und die den Betrieb des Herzstimulators 10 beeinflussen.

Fig. 3 zeigt außerdem noch, dass für den Fall, dass der Herzstimulator 10 ein Defibrillator ist, auch noch ein rechtsventrikulärer Schockgenerator RV-SCHOCK 62 und ein linksventrikulärer Schockgenerator LV-SCHOCK 64 vorgesehen sein können, die jeweils einerseits mit einem elektrischen Anschluss RV-COIL für die rechtsventrikuläre Schockelektrode bzw. einem Anschluss LV-COIL für die linksventrikuläre Schockelektrode verbunden sein kann sowie andererseits mit der Steuereinheit CTRL 54.

Die Stimulationseinheiten 50 und 52 sowie die Schockgeneratoren 62 und 64 stellen jeweils Therapieeinheiten des implantierbaren medizinischen Gerätes 10 dar, die durch die Steuereinheit 54 gesteuert werden. Die ggf. bedarfsabhängige und ratenangepasste Abgabe von Stimulationsimpulsen an eine oder mehrere Herzkammern sowie die Abgabe von Kardioversionsimpulsen oder Defibrillationsschocks im Falle von Tachykardien oder Fibrillationen sind grundsätzlich bekannt und werden von der Steuereinheit 54 gesteuert. Derartige Therapien können jedoch durch die Steuereinheit 54 weiter optimiert werden, indem die Steuereinheit 54 diese Therapien in Abhängigkeit des Akzelerometerausgangssignals aus dem Beschleunigungssensor 80 und/oder von einer Beschleunigungsauswerteeinheit 84 aus dem Akzelerometerausgangssignal abgeleiteter Signalkomponenten und Größen steuert.

Der Speicher MEM 56 ist ausgangsseitig mit der Telemetrieeinheit TEL 58 verbunden, die so ausgebildet ist, dass die in dem Speicher 56 jeweils gespeicherten Werte zu einem regelmäßig wiederkehrenden Sendezeitpunkt von der Telemetrieeinheit 58 mittels einer der Telemetrieeinheit 58 zugeordneten Sendeeinheit derart ausgesandt werden, dass die entsprechenden Werte von einem externen Gerät 96 empfangen und beispielsweise an ein Servicecenter, einen Arzt oder dergleichen weitergeleitet werden können.

Die Steuereinheit 54 ist außerdem mit einem 3D-Beschleunigungssensor (3D-Akzelerometer) verbunden, der die externe Beschleunigungssensoreinheit 80 (siehe Figur 2) aufweist, die zur Positionierung in der Pulmonalarterie ausgebildet ist, um dort mehrachsig Beschleunigungen zu erfassen. Die externe Beschleunigungssensoreinheit 80 ist über die Anschlussleitung 82 mit der Beschleunigungs-Auswerteinheit 84 verbunden und stellt mit dieser zusammen den 3D-Beschleunigungssensor (das 3D-Akzeleromter) dar. Daneben kann der 3D-Beschleunigungssensor dazu ausgebildet sein, dynamische Beschleunigungen, z.B. bei körperlicher Aktivität, zu erfassen oder auch eine jeweilige Gerätelage, die im implantierten Zustand des Gerätes einer jeweiligen Körperlage entspricht.

Die Beschleunigungs-Auswerteeinheit 84 ist ausgebildet, von der Beschleunigungssensoreinheit 80 im Bereich der Pulmonalarterie erfasste mehrachsige Beschleunigungen widerspiegelnde Akzelerometerausgangssignale auszuwerten, wie weiter unten näher erläutert ist.

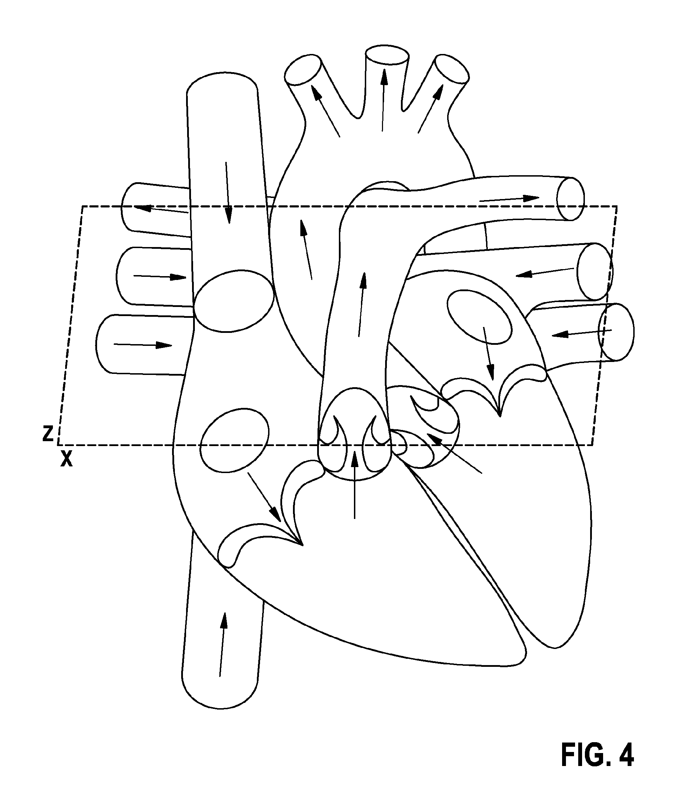

Die Figuren 4, 6 und 9 zeigen, wie im Bereich der Verzweigung des Truncus pulmonalis in die zwei Arteriae pulmonales die Pulmonalarterie (PA) Seite an Seite mit der Aorta (Ao) liegt. Bewegungen dieser Gefäße wie auch Herztöne, die dem pulsatilen Blutstrom aus den Herzkammern folgen, übertragen sich hier auch auf das jeweils andere Gefäß. Diese Signale werden mit dem 3D-Akzelerometer 80 aufgezeichnet. Bevorzugt ist die Platzierung der externen Sensoreinheit 80 in der Pulmonalarterie, da der Einfluss der Aorta in der Pulmonalarterie wohl deutlicher zu erkennen ist als der Einfluss der Pulmonalarterie in der Aorta.

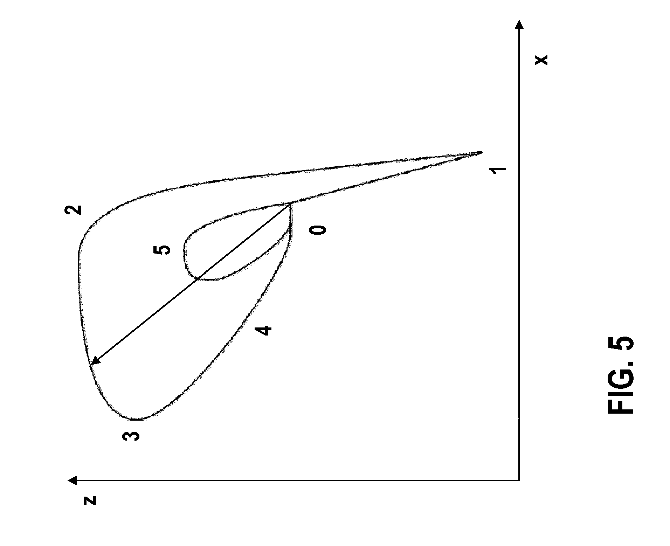

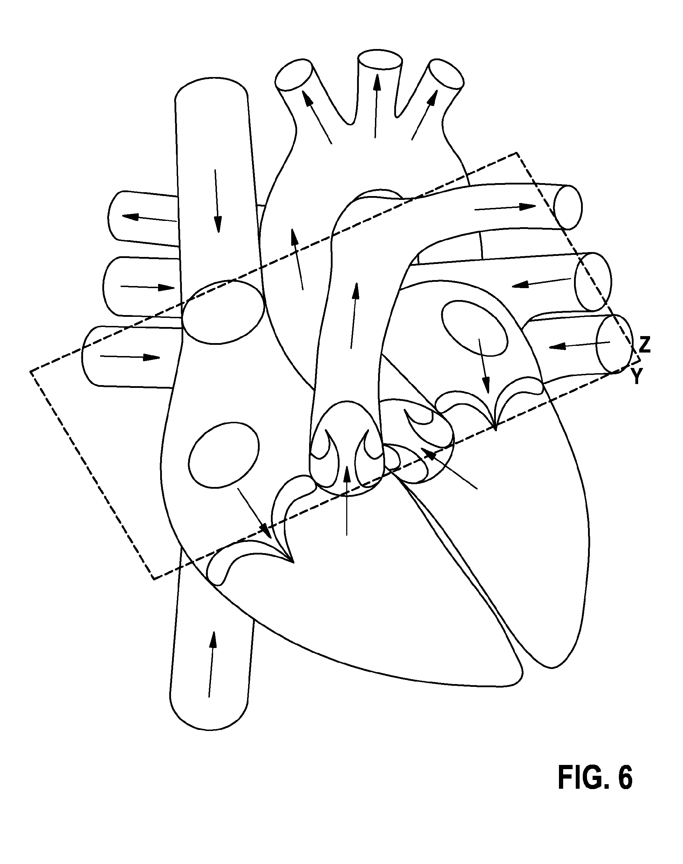

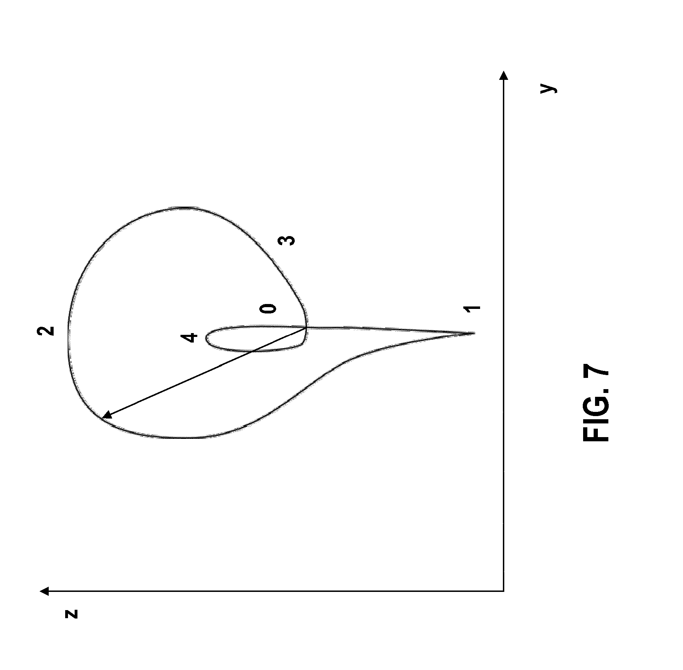

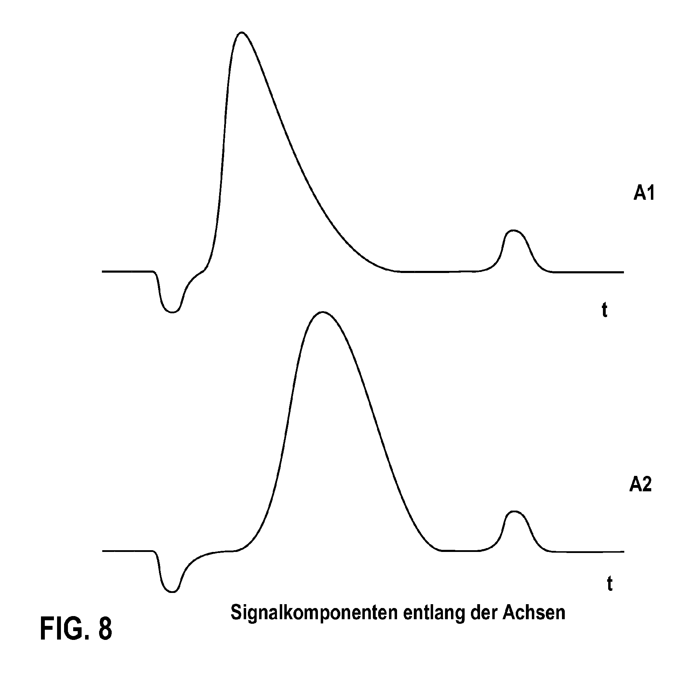

Figuren 5 und 7 zeigen die zu erwartenden Richtungskomponenten des durch die Blutpulsation verursachten Akzelerometerausgangssignals. Figur 8 zeigt den sich hieraus ergebenden Verlauf des Beschleunigungssignals in Richtung der x- bzw. y-Achse entsprechend den Figuren 4 und 6. Das obere Signal A1 entspricht der rechts-, das untere Signal A2 der linksventrikulären Kontraktion.

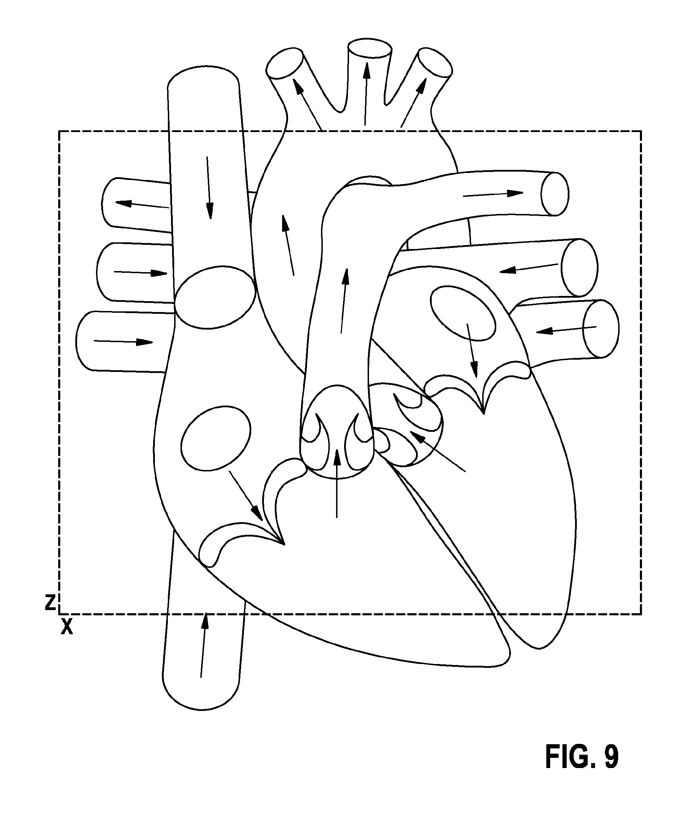

Figure 9 zeigt den Einfluss der Gravitation auf das Akzelerometerausgangssignal.

Aufgrund der unterschiedlichen Anatomie von Aorta und Pulmonalarterie sind die Richtungskomponenten des durch die Blutpulsation verursachten Akzelerometerausgangssignals unterschiedlich. Des Weiteren entsteht durch die typische Verzögerung zwischen den Kontraktionen von linkem und rechtem Ventrikel ein Zeitversatz zwischen den Komponenten. Diese beiden Effekte erlauben die Trennung von Beschleunigungskomponenten aus der Aorta und der Pulmonalarterie.

Im Folgenden werden die zu erwartenden Signalkomponenten anhand der schematischen Darstellungen in Figuren 4 bis 9 näher erläutert.

Während eines Herzzyklusses sind die folgenden, sich überlagernden Komponenten im Akzelerometersignal zu erwarten.

Zunächst zur Pumpmechanik des Herzens und dem daraus resultierenden Blutfluss.

Das dreidimensionale Signal, das aus der An- und Entspannung des Herzens sowie des Blutflusses entsteht, lässt sich in zwei Komponenten aufteilen, nämlich:
(1) eine erste Signalkomponente, die der rechtsventrikulären Kontraktion entspricht und die in der Ebene verläuft, die durch den rechtsventrikulären Ausflusstrakt und die rechte Pulmonalarterie definiert wird (siehe Figur 4); und
(2) eine zweite Signalkomponente, die aus der Linksherzkontraktion entspringt und in der Ebene verläuft, in der der Aortenbogen liegt (siehe Figur 6).

Die z-Achsen fallen näherungsweise zusammen, x- und y-Achse stehen in etwa senkrecht aufeinander.

Der zeitliche Verlauf des Beschleunigungsvektors in Figur 5 wird durch die Kontraktion des rechten Herzens verursacht. Aus der Ruhelage 0 wird bei Kontraktion des rechten Ventrikels der gesamte Bereich der Herzbasis mitsamt den Arterien in Richtung Herzspitze gezogen (1). Daraufhin strömt Blut aus dem Ventrikel in den Truncus pulmonalis und trifft auf die Verzweigung der beiden Pulmonalarterien (2). Der Blutdruckpuls erreicht die rechte Pulmonalarterie, sodass der Akzelerometer entlang der Achse der PA beschleunigt wird (3). Das Herz beginnt sich zu entspannen und das Akzelerometersignal kehrt zum Ursprung zurück (4). Mit der Entspannung des Ventrikels hebt sich die Ventilebene wieder Richtung Herzbasis, sodass die PA von der Herzspitze weggedrückt wird (5).

Der zeitliche Verlauf des Beschleunigungsvektors in Figur 7 wird durch die Kontraktion des linken Herzens verursacht. Aus der Ruhelage 0 wird bei Kontraktion des linken Ventrikels der Bereich der Herzbasis samt Aorta in Richtung Herzspitze gezogen (1). Daraufhin strömt Blut in den Aortenbogen; das Akzelerometer folgt der Wellenfront und beschreibt einen Bogen (2), bis es in die Ruhelage 0 zurückkehrt (3). Mit der Entspannung des Ventrikels hebt sich die Ventilebene wieder in Richtung Herzbasis, sodass die Aorta von der Herzspitze weggedrückt wird (4).

Die Komponenten entlang der x- und y-Achse könnten wie in Figur 8 dargestellt aussehen. Der geringe Einbruch zu Beginn des Zyklus' (1) entspringt der Bewegung der Ventilebene und dürfte bei beiden Signalen synchron ablaufen. Gleiches gilt für die Entspannung, die zu einer Welle am Ende des Zyklus' führt (5 bzw. 4). Die Austreibung beginnt im rechten Ventrikel (2 und 3) aufgrund des geringeren Gegendrucks etwas früher als in der linken Kammer (2). Aufgrund Bogenform der Aorta und der Dämpfung (im Falle einer Platzierung des Akzelerometers in der Pulmonalarterie) dürfte der Beschleunigungspeak, der der Linksherzkontraktion (unteres Signal) entspricht, breiter als der der Rechtsherzkontraktion sein.

Nun zum Einfluss der Gravitation; siehe Figur 9.

Die Gravitation geht als zeitlich konstante Komponente in das Akzelerometersignal ein. Diese Komponente kann zur Kalibration der Orientierung des Akzelerometers im Raum und, bei bekannter Position des Patienten (liegend/stehend), zur Kalibration der Orientierung des Akzelerometers im Körper verwendet werden. Das ist z.B. dann nötig, wenn die Richtung eines Peaks als Kriterium für die Zuordnung zu einer der Signalkomponenten A1 bzw. A2 verwendet wird. Die Kalibration ist bei starken Störeinflüssen schwierig, z.B. bei hoher körperlicher Aktivität des Patienten; Methoden, bei denen die Kalibration unumgänglich ist, müssten sich evtl. auf Zeiten beschränken, in denen die Störungen minimal sind, z.B. nachts.

Nun zu möglichen Störeinflüssen.

Störeinflüsse entstehen durch Geräterauschen, körperliche Aktivität des Patienten oder Bewegungen außerhalb des Körpers (Bus/Bahn/Auto, Aufzug, Rolltreppe etc.). Aussehen, Amplitude und räumliche Orientierung hängen von den Ursachen ab. Geräterauschen ist ein für alle Raumrichtungen identisches weißes Rauschen. Ähnlichkeit zu diesem werden Akzelerometersignale haben, die von Vibrationen von Verkehrsmitteln verursacht werden, allerdings räumlich gerichtet in Richtung der Gravitation und evtl. mit einigen stärker ausgebildeten Frequenzbereichen. Richtungswechsel, Anfahren und Abbremsen (beim Autofahren, in Fahrstühlen oder auf Rolltreppen etc.) treten als temporäre gerichtete Peaks auf.

Nun zur Nutzung des Akzelerometerausgangssignals.

Aus den Einzelkomponenten des Akzelerometerausgangssignals lassen sich diagnostische Indizes bestimmen: Amplituden zu ausgewählten Zeitpunkten, Zeitdifferenzen zwischen ausgewählten Ereignissen etc. Insbesondere ist es zu erwarten, dass die Amplitude der jeweiligen Beschleunigungskomponente proportional zur Kontraktilität des jeweiligen Ventrikels ist; aus geeigneten Merkmalen der Links- bzw. Rechtsherzkomponente lässt sich das AV-Delay bestimmen. Die Beschleunigungsauswerteeinheit 84 ist vorzugsweise dazu ausgebildet, nicht nur die Signalkomponenten zu bestimmen, sondern aus diesen weitere Größen und insbesondere die genannten diagnostischen Indizes abzuleiten.

Als Erweiterung der vorgestellten Idee kann die Beschleunigungsauswerteeinheit 84 auch ausgebildet sein, die diagnostischen Indizes auch in Abhängigkeit von der Aktivität des Patienten auszuwerten. Dazu extrahiert die Beschleunigungsauswerteeinheit diejenige Signalkomponente aus dem Akzelerometerausgangssignal, die die Aktivität repräsentiert.

In der gleichen Weise kann die Beschleunigungsauswerteeinheit eine Auswertung der Indizes in Abhängigkeit der Herzrate vornehmen, die die Beschleunigungsauswerteeinheit 84 ebenfalls mit bekannten Methoden aus dem Akzelerometerausgangssignal bestimmt.

Das beschriebene Sensorsystem lässt sich mit weiteren Sensoren kombinieren, so dass die beschriebenen Indizes auch in Abhängigkeit von anderen Größen, z.B. Blutdruck oder Impedanzsignalen, ausgewertet werden.

Ergänzt wird die Monitoring-Funktionalität der vorgestellten Idee durch die getrennte Bestimmung der Klappenöffnungs- und -verschlusszeiten. Veränderungen in den charakteristischen Signalverläufen können auf das Entstehen/Vorhandensein von Verengungen bzw. Undichtigkeiten an den Ein-/Ausstromklappen der beiden Ventrikel hinweisen.

Außerdem wird die dynamische Ausdehnung der Aorta während der linksseitigen Systole indirekt erfasst. Somit können quantitative Rückschlüsse über die Elastizität der Aorta gewonnen werden. Auch hier ist eine Detektion krankhafter Veränderungen (Aneurysma, Stenose, Arteriosklerose) durch die Analyse des Akzelerometerausgangssignals möglich.

Strukturelle Veränderungen im Ventrikel werden durch getrennte Analyse der Änderung der Länge und der Drehbewegung, die der Ventrikel während der Systole durchläuft, erkannt und quantifiziert.

Ausgeprägte paradoxe Septumbewegungen können im Akzelerometerausgangssignal durch entsprechende Filterung isoliert werden.

Des Weiteren lässt sich die Atmung überwachen: Das Heben und Senken des Diaphragmas wird als niederfrequentes Signal aus dem Akzelerometerausgangssignal extrahiert. Ferner schwanken aufgrund der Atmung die Volumenverteilung im Thorax sowie die Orientierung des Herzens, wodurch ein Atemsignal rekonstruiert werden kann.

Eine weitere Anwendung ist die hämodynamische Charakterisierung von Arrhythmien, u. a. als zusätzliches Kriterium für das Auslösen eines Defibrillationsschocks.

Das vorgestellte System kann auch als hämodynamischer Sensor angewendet werden, indem die Beschleunigungsauswerteeinheit 84 aus den Signalkomponenten A1 und A2 geeignete Parameter bestimmt, die die Leistung des Herzens beschreiben. Diese hämodynamischen Parameter können zum Monitoring einer Herzinsuffizienz oder zur Optimierung von Therapieparametern (z.B. AV-, VV-Delay) verwendet werden.

In Kombination mit anderen Signalen, die z.B. die elektrische Aktivität des Herzens beschreiben und die beispielsweise von den entsprechenden Sensingeinheiten gewonnen werden können (IEGM, EKG), kann die Beschleunigungsauswerteeinheit Parameter bestimmen, die die elektrisch-mechanische Kopplung beschreiben. Auch diese Parameter können zum Verlaufsmonitoring einer Erkrankung oder zur Therapieoptimierung verwendet werden.

Das vorgestellte System benötigt nur einen einzigen Sensor, um das Kontraktionsverhalten der Herzens zu messen und die Funktion von linkem und rechtem Ventrikel zu unterscheiden und zu vergleichen. Damit kann es mehr leisten als Sensoren, die an anderen Stellen des Körpers platziert werden. Im Vergleich zu einem 2-Sensor-System sind Energiebedarf, Fehleranfälligkeit sowie die Belastung für Arzt und Patient deutlich verringert.

Ein Beschleunigungssensor in direkter Herznähe bietet zusätzliche Monitoring-Möglichkeiten. Die Technologie könnte sowohl für Patienten mit Schrittmacher/ICD als auch für HF-Patienten, die noch keine sonstigen Implantate haben, Anwendung finden (Monitoring-Implantat).

Mit den vorgestellten Realisierungen deckt das System einen weiten Bereich von Anwendungen ab. Neben der Integration in Prädiktionsalgorithmen oder zur Anpassung medizinischer Geräte lassen sich auch Kenngrößen ableiten und Krankheiten detektieren/überwachen, die medizinisch geschultem Personal bekannt sind (Verhältnis von Kontraktilitäten, Atemparameter, Verzögerungszeiten zwischen linkem und rechtem Ventrikel etc.; Arrhythmien, Klappendefekte, Kontraktilitätseinbußen, paradoxe Septumsbewegungen etc.).

## Patentansprüche

1. Implantierbares medizinisches Gerät mit einem mehrachsigen Beschleunigungssensor und einer mit diesem verbundenen Auswerteeinheit, **dadurch gekennzeichnet, dass** die Auswerteinheit ausgebildet ist, ein Akzelerometerausgangssignal des Beschleunigungssensors auszuwerten, indem die Auswerteeinheit
das Akzelerometerausgangssignal in zumindest zwei Signalkomponenten aufspaltet, von denen wenigstens zwei in Zusammenhang mit einer rechts- bzw. linksventrikulären Kontraktion eines Herzens stehen;
in den wenigstens zwei Signalkomponenten Ereignisse detektiert und/oder Signalmerkmale bestimmt; und
zumindest eine Kenngröße K unter Auswertung der wenigstens zwei Signalkomponenten und der aus diesen bestimmten Ereignisse und/oder Signalmerkmale bestimmt.

2. Implantierbares medizinisches Gerät nach Anspruch 1, **dadurch gekennzeichnet, dass** der Beschleunigungssensor ein 3D-Akzelerometer ist.

3. Implantierbares medizinisches Gerät nach Anspruch 1 oder 2, **dadurch gekennzeichnet, dass** die Auswerteeinheit ausgebildet ist, die wenigstens zwei Signalkomponenten anhand von Richtungskomponenten des Akzelerometerausgangssignal zu bestimmen.

4. Implantierbares medizinisches Gerät nach einem der Ansprüche 1 bis 3, **dadurch gekennzeichnet, dass** die Kenngröße K das Verhältnis zwischen zwei Amplitudenwerten als jeweiliges Signalmerkmal aus den wenigstens zwei Signalkomponenten ist, insbesondere das Verhältnis der Maximalamplituden innerhalb eines Herzzyklus.

5. Implantierbares medizinisches Gerät nach einem der Ansprüche 1 bis 4, **dadurch gekennzeichnet, dass** die Kenngröße K den Zeitversatz zwischen zwei einander entsprechenden Ereignissen in den wenigstens zwei Signalkomponenten ist, insbesondere eine Größe, die mit einer interventrikulären Verzögerungszeit (VV-Delay) zusammenhängt.

6. Implantierbares medizinisches Gerät nach einem der Ansprüche 1 bis 5, **dadurch gekennzeichnet, dass** die Auswerteeinheit zur Ableitung zumindest einer weiteren Signalkomponente A3 und Bestimmung der Kenngröße K in Abhängigkeit eines aus der Signalkomponente A3 abgeleiteten Wertes ausgebildet ist, wobei die Signalkomponente A3 vorzugsweise eine die Aktivität des Patienten widerspiegelnde Signalkomponente und der aus ihr abgeleitete Wert die mittlere Aktivität in einem vordefinierten Zeitintervall ist.

7. Implantierbares medizinisches Gerät nach einem der Ansprüche 1 bis 5, **dadurch gekennzeichnet, dass** die Auswerteeinheit ausgebildet ist, zusätzlich eine Herzrate aus den Signalkomponenten A1 und/oder A2 abzuleiten und die Kenngröße K in Abhängigkeit der Herzrate zu bestimmen.

8. Implantierbares medizinisches Gerät nach einem der Ansprüche 1 bis 7, **dadurch gekennzeichnet, dass** das implantierbare Gerät zusätzlich mit einem Blutdruck- und/oder Impedanzsensor ausgestattet ist und die Auswerteinheit zur Bestimmung der Kenngröße K in Abhängigkeit einer aus dem Blutdruck und/oder der Impedanz abgeleiteten Größe ausgebildet ist.

9. Implantierbares medizinisches Gerät nach einem der Ansprüche 6 bis 8, **dadurch gekennzeichnet, dass** die Auswerteinheit ausgebildet ist, die Kenngröße K nur zu solchen Zeitpunkten zu bestimmen, in denen eine aus der Signalkomponente A3 oder der Herzrate oder dem Blutdruck oder der Impedanz abgeleitete Größe in einem vordefinierten Wertebereich liegt.

10. Implantierbares medizinisches Gerät nach einem der Ansprüche 1 bis 9, **dadurch gekennzeichnet, dass** das Gerät dazu ausgebildet ist, Kenngrößen der Atmung zu bestimmen, insbesondere Frequenz und Amplitude.

11. Implantierbares medizinisches Gerät nach einem der Ansprüche 1 bis 10, **dadurch gekennzeichnet, dass** das implantierbare Gerät eine Steuereinheit aufweist, die ausgebildet ist, die Kenngröße K zur Detektion von Arrhythmien zu verwenden und Therapieeinheiten des implantierbaren medizinischen Gerätes entsprechend zu steuern.

12. Implantierbares medizinisches Gerät nach einem der Ansprüche 1 bis 11, **dadurch gekennzeichnet, dass** das implantierbare Gerät eine Steuereinheit aufweist, die ausgebildet ist, in Abhängigkeit der Kenngröße K Einstellungen des implantierbaren medizinischen Geräts anzupassen.

13. Implantierbares medizinisches Gerät nach einem der Ansprüche 1 bis 12, **dadurch gekennzeichnet, dass** das implantierbare Gerät ein implantierbarer biventrikulärer Herzschrittmacher und/oder Defibrillator/Kardioverter ist.

14. Verfahren zur Herzüberwachung mit den Verfahrensschritten:
(a) Aufspaltung des Akzelerometerausgangssignals A in zumindest zwei Signalkomponenten A1 und A2, die in einem Zusammenhang mit einer rechts- bzw. einer linksventrikulären Kontraktion eines Herzens stehen;
(b) Bestimmung geeigneter Ereignisse und/oder Merkmale aus den Signalkomponenten A1 und A2; und
(c) Bestimmung zumindest einer Kenngröße K unter Verwendung mindestens der Signalkomponenten A1 und A2 und der in (b) bestimmten Ereignisse und/oder Merkmale.
